# EUROPEAN PATENT APPLICATION

(11) **EP 2 532 666 A1**
(43) Date of publication of application: **12.12.2012**
(21) Application number: 11739347.0
(22) Date of filing: 07.01.2011
(51) Int. Cl.: C07D 487/04, A61K 31/55, A61P 35/00

(54) **PHARMACEUTICALLY ACCEPTABLE SALTS OF PYRROLO-NITROGENOUS HETEROCYCLIC DERIVATIVES, PREPARATION METHOD AND MEDICAL USE THEREOF**

(30) Priority: 04.02.2010 CN 201010109068
(71) Applicant: Jiangsu Hengrui Medicine Co., Ltd., Lianyungang, Jiangsu 222002 (CN); Shanghai Hengrui Pharmaceutical Co. Ltd., Minhang District Shanghai 200245 (CN)
(72) Inventor: TANG, Peng Cho, Shanghai 200245 (CN)
(74) Representative: Pistolesi, Roberto
(86) International application number: PCT/CN2011/070076
(87) International publication number: WO 2011/095068

(57) **Abstract**

Pharmaceutically acceptable salts of pyrrolo-nitrogenous heterocyclic derivatives, preparation method and medical use thereof are disclosed. More specifically, pharmaceutically acceptable salts of (*R,Z*)-2-(5-fluoro-2-oxo-1,2-dihydro-indol-3-methylene)-5-(2-hydroxy-3-morpholinyl-4-yl-propyl)-3-methyl-5,6,7,8-tetrahydro-1*H-*pyrrolo[3,2-*c*]azepin-4-one presented by formula (I), the preparation method and the use thereof as therapeutic agents, especially as protein kinase inhibitors, are disclosed.

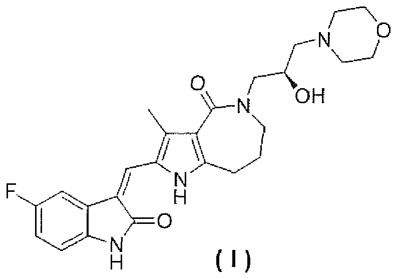

## Description

### FIELD OF THE INVENTION

The present invention relates to pyrrolo-nitrogenous heterocyclic derivatives pharmaceutical salts, preparation processes and pharmaceutical use thereof. Specifically, the present invention relates to (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4-one pharmaceutical salts, preparation processes and the use as therapeutic agents, particularly as protein kinase inhibitors.

### BACKGROUND OF THE INVENTION

Signal transduction is a fundamental regulation mechanism whereby extracellular stimuli are relayed to the interior of cells. These signals regulate a wide variety of physical responses in the cell including proliferation, differentiation and apoptosis. Protein kinases (PKs) play critical roles in these processes. There are two classes of protein kinases (PKs): the tyrosine kinases (PTKs) and the serine/threonine kinases (STKs). PTKs can phosphorylate tyrosine residue on a protein. STKs can phosphorylate serine and threonine residue. Tyrosine kinases can be divided into either the receptor-type (receptor tyrosine kinase, RTKs) or the non-receptor type (non-receptor tyrosine kinase).

The receptor tyrosine kinases (RTKs) family can be divided into many subtribes, which mainly include: (1) the ErbB (Her) family such as the EGFR(Her-1), Her-2, Her-3 and Her-4; (2) the insulin receptor family such as the insulin receptor (IR), insulin-like growth factor-I receptor (IGF-IR) and the like; (3) the Class III family such as the platelet-derived growth factor receptor (PDGFR), the stern cell factor SCFR (c-Kit) and the like. Otherwise, hepatocyte growth factor receptor (HGFR) c-Met and vascular endothelial growth factor receptor (VEGFR) also belong to RTKs family. They play critical roles in the regulation of cell proliferation and differentiation as signal messenger (Schlessinger and Ullrich, Neuron 1992, 9, 383).

EGFR subtribe is one of the important members of the RTKs family. These RTKs can be activated by ligand-mediated homodimerization or heterodimerization among receptors. Dimerization results in phosphorylation of tyrosine residues in intracellular catalytic domain of the receptors, producing a future binding site for the subsequent signal molecules. This is followed by the activation of intracellular signaling pathways such as those involving the microtubule associated protein kinase (MAP kinase) and the phosphatidylinositol3-kinase (PI-3 kinase), leading to cell signal response ultimately. It has been identified that such mutated and overexpressed forms of tyrosine kinases, like EGFR and/or Her-2, are present in a large proportion of common human cancers such as breast cancer, prostate cancer, non-small cell lung cancer, gastrointestinal cancer, ovarian cancer and pancreatic cancer and the like. Thus the prevalence and relevance of tyrosine kinase are confirmed in the oncogenesis and cancer growth.

As the Class III family of receptor tyrosine kinases, the platelet derived growth factor receptor (PDGFR) and c-Kit, transmit signals after activation through dimerization, which is similar to the ErbB family. The members of this family are closely related to the differentiation, proliferation and migration of tumor cell, as well the angiogenic process. For Example, a high expression or mutation of c-Kit could be found in small cell bronchial carcinoma, melanoma, breast cancer and neuroblastoma (see Schũtte et al., innovartis 3/2001). Mutations can lead to sustained activation of c-Kit receptor, especially in gastrointestinal stromal tumor (GIST), and lead to a high cell division rate and possibly genomic instability. Thus cancer is induced [see Weber et al., J. Clin. Oncol. 22(14S), 9642 (2004)].

Another important member of RTKs is the vascular endothelial growth factor receptor (VEGFR). VEGFR is directly involved in angiogenesis and can induce proliferation and migration of endothelial cell, which subsequently leads to the formation of capillary tubes that promote the formation of the hyperpermeable, immature vascular network which nourishs cancer growth. In addition to its angiogenic activity, VEGFR and VEGF may promote tumor growth directly by pro-survival effects in tumor cells. It was observed that VEGFR is highly expressed in a variety of solid malignant tumors, such as lung carcinoma, breast carcinoma, ovarian carcinoma, pancreatic cancer and melanoma. Therefore, the development of tumors can be inhibited by inhibiting VEGFR activity. That is beneficial in the treatment of tumors.

In addition, as one member of the RTKs, it was proved that the hepatocyte growth factor receptor c-Met (HGFR) are closely related to oncogenesis, invasion and metastasis of tumor, as well as to the enhancement of cell motility (see, Ma, P.C. et al. (2003b). Cancer Metastasis Rev, 22, 309-25; Maulik, G. et al. (2002b). Cytokine Growth Factor Rev, 13, 41-59).

The main characteristics of cancer are genome damage and uncontrolled signal pathways. Genomic damage leads to changing or losing biological function of key regulating proteins, and then damages the signal transduction pathways. The aberrant signal pathways make cancer cells live and proliferate continuously in the state of genetic damage. As the foundation of achieving these regulating progress, PTKs are closely related to oncogenesis and tumor growth, and became the important target for treating tumor. It is expected to ameliorate or treat physiological disorders produced by cell non-normal proliferation mediated by RTKs effectively through inhibiting one or more of the RTKs.

WO2008/138232 disclosed a novel kind of pyrrolo-nitrogenous heterocyclic derivatives and the use as protein kinase inhibitors thereof, wherein the compound disclosed in Example 53 is (*R*,*Z*)-2-(5-(fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4-one of formula (1).

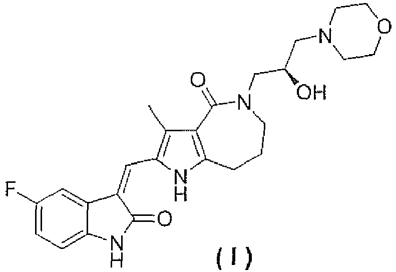

The inventor finds that the compound of formula (I) is poorly soluble in conventional solvents and thus disadvantageous to be prepared into a medicinal dosage form, limiting their in vivo bioavailability. It is necessary to develop new forms of the compound of formula (I) to improve its solubility and pharmacokinetic absorption, which can be used in conventional preparation of dosage forms.

The present invention is directed to provide pharmaceutically acceptable salts of the compound of formula (I), thereby improve their physical/chemical properties and pharmacokinetic characteristics.

### DESCRIPTION OF THE INVENTION

The present invention relates to the pharmaceutically acceptable salts of the compound of formula (I), and the preparation methods thereof. Preferably, the maleate salt of the compound of formula (I) has advantages in solubility, bioavailability and pharmacokinetics compared with the compound of formula (I) itself and its other salts.

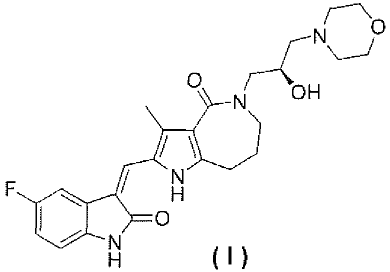

In the first aspect, the present invention relates to the pharmaceutically acceptable salts of (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4-one of formula (I), wherein said salts are conventional inorganic salts or organic salts in the art. Further, said inorganic salts are selected from the group consisting of hydrochloride, hydrobromide, sulfate, nitrate or phosphate, preferably hydrochloride; said organic salts are selected from the group consisting of mesylate, maleate, tartrate, succinate, acetate, trifluoroacetate, fumarate, citrate, benzene sulfonate, benzoate, naphthalene sulphonate, lactate and malate, preferably malate, lactate, mesylate or maleate. Especially the maleate salt of the compound of formula (I), which has advantages in solubility, bioavailability and pharmacokinetics compared with the compound of formula (I) itself and its other salts.

In the second aspect, the present invention relates to the preparation method for the pharmaceutically acceptable salts of (*R*,*Z*)-2-(5-fluoro-2-oxo-1,2-dihydro-indol-3-ylidenemeth yl)-5-(2-hydroxy-3-morpholin-4-yl-propyl)-3-methyl-5,6,7,8-tetrahydro-1*H*-pyrrolo[3,2-c]aze pin-4-one of formula (I), and the salts can be prepared according to conventional salt formation methods in the art. Specifically, said method comprises the step of reacting (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4-one with a corresponding acid to prepare the salt, wherein said acid is an inorganic acid/organic acid selected from the group consisting of phosphoric acid, hydrochloric acid, sulfuric acid, nitric acid, hydrobromic acid, methanesulfonic acid, maleic acid, tartaric acid, succinic acid, acetic acid, trifluoroacetic acid, fumaric acid, citric acid, benzenesulfonic acid, benzoic acid, naphthalenesulfonic acid, lactic acid and malic acid.

In the third aspect, the present invention relates to a pharmaceutical composition comprising a therapeutically effective amount of the pharmaceutically acceptable salts of (*R*,*Z*)-2-(5-fluoro-2-oxo-1,2-dihydro-indol-3-ylidenemethyl)-5-(2-hydroxy-3-morpholin-4-y]-propyl)-3-methyl-5,6,7,8-tetrahydro-1*H*-pyrrolo[3,2-c]azepin-4-one of formula (I), and pharmaceutically acceptable carriers thereof.

In the fourth aspect, the present invention relates to a use of pharmaceutically acceptable salts or pharmaceutical compositions of (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H-*pyrrolo[3,2-*c*]azepin-4-one of formula (I) in the preparation of a medicament for the treatment of protein kinases related diseases, wherein said protein kinases related diseases are selected from the group consisting of diseases related with VEGFR-2, EGFR, HER-2, PDGFR, c-Kit, c-Met and FGFR. Wherein said diseases are cancers selected from the group consisting of lung cancer, breast cancer, epidermal squamous cell carcinoma and gastric cancer.

In the fifth aspect, the present invention relates to a method for the treatment of protein kinases related diseases, comprising administrating the subject in need a therapeutically effective amount of pharmaceutically acceptable salts of (*R*,*Z*')-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4-one of formula (I) and pharmaceutical compositions thereof.

In the sixth aspect, the present invention relates to a use of pharmaceutically acceptable salts of (*R*,*Z*)-2-(5-fluoro-2-oxo-1,2-dihydro-indol-3-ylidenetnethyl)-5-(2-hydroxy-3-morpholin-4-yl-propyl)-3-methyl-5,6,7,8-tetrahydro-1*H*-pyrrolo[3,2-c]azepin-4-one of formula (I) or pharmaceutical compositions thereof in the preparation of protein kinase inhibitor drugs, wherein said protein kinase is selected from the group consisting of VEGFR-2, VEGFR, HER-2, PDGFR, c-Kit, c-Met and FGFR.

In the seventh aspect, the present invention relates to a use of pharmaceutically acceptable salts of (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2,-*c*]azepin-4-one of formula (I) or pharmaceutical compositions for use as a medicament for the treatment of protein kinases related diseases, wherein said diseases are cancer selected from the group consisting of lung cancer, breast cancer, epidermal squamous cell carcinoma and gastric cancer.

It has been identified through experiment results that maleate of the compound of formula (I) has better solubility, bioavailability and pharmacokinetics than the compound of formula (I) itself and other salts thereof.

### SYNTHESIS METHOD OF THE COMPOUND OF FORMULA (I) (KEY SATARTING MATERIAL) IN THIS INVENTION

The preparation method of (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin n -4-one of formula (I) is according to the Example 53 disclosed by WO2008/138232, therefore this disclosed content is incorporated by reference.

### SPECIFIC IMPLEMENTION METHODS

The present invention is further described by the following Examples which are not intended to limit the scope of the invention.

### EXAMPLES

The structures of all compounds were identified by nuclear magnetic resonance (NMR) and/or mass spectrometry (MS). NMR chemical shifts (δ) were recorded as ppm (10⁻⁶) . NMR was performed on a Bruker AVANCE-400 spectrometer. The detective solvent is deuterated-dimethyl sulfoxide (*d*-DMSO) with tetramethylsilane (TMS) as the internal standard, and chemical shifts were recorded as ppm (10⁻⁶).

MS was determined on a FINNIGAN LCQAd (ESI) mass spectrometer (Thermo, Model: Finnigan LCQ advantage MAX).

HPLC was determined on an Agilent 1200DAD high pressure liquid chromatography spectrometer (Sunfire C18 150×4.6 mm chromatographic column) and a Waters 2695-2996 high pressure liquid chromatography spectrometer (Gimini C18 150×4.6 mm chromatographic column).

Column chromatography generally used Yantai Huanghai 200∼300 mesh silica gel as carrier.

The starting materials of the present invention were known and can be purchased from ABCR GmbH & Co. KG, Acros Organics, Aldrich Chemical Company and so on. Or, they can be prepared by the conventional synthesis methods in the prior art.

Unless otherwise stated, the following reactions were placed under argon atmosphere or nitrogen atmosphere.

The term "argon atmosphere" or "nitrogen atmosphere" refers to that a reaction flask was equipped with a balloon filled about 1 L nitrogen.

The term "hydrogen atmosphere'' refers to that a reaction flask was equipped with a balloon filled about 1 L hydrogen.

Unless otherwise stated, the solution used in Examples refers to an aqueous solution. Unless otherwise stated, the reaction temperature was room temperature.

Room temperature was the most proper reaction temperature, which was 20 °C-30 °C.

The reaction processes of the Examples were monitored by thin layer chromatography (TLC). The developing solvent systems comprised dichloromethane and methanol system, n-hexane and ethyl acetate system, petroleum ether and ethyl acetate system, and acetone. The ratio of the volume of the solvent was adjusted according to the polarity of the compounds.

The elution systems of column chromatography comprised: A: dichloromethane, methanol and acetone system; B: hexane and ethyl acetate system. The ratio of the volume of the solvent was adjusted according to the polarity of the compounds, and sometimes a small amount of ammonia and acetic acid can also be added.

### Example 1

### (R,Z)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1H-pyrrol[3,2,-c]azepin-4-one maleate

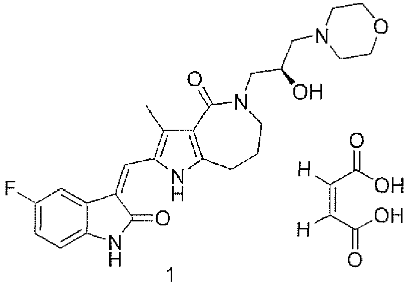

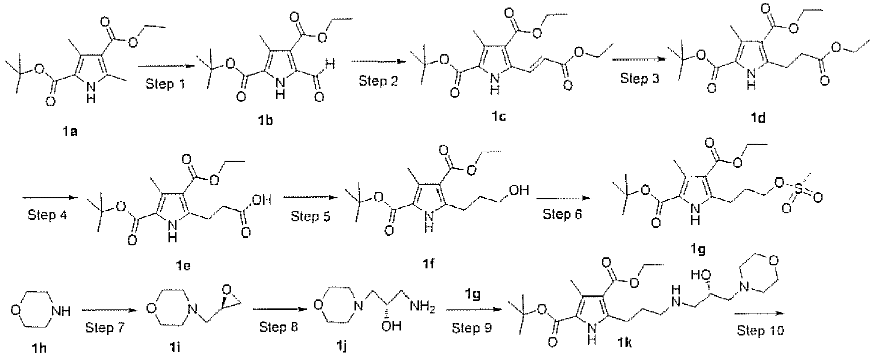

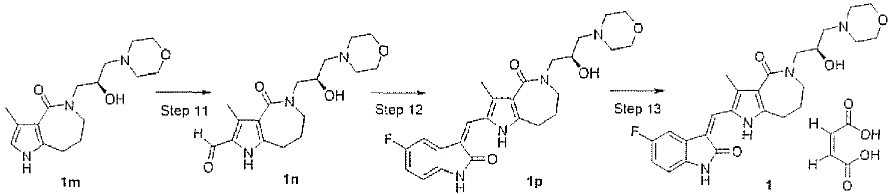

### Step 1

### 5-Formy]-3-methyl-1H-pyrrole-2,4-dicarboxylic acid 2-tert-butyl ester 4-ethyl ester

3,5-Dimethyl-1*H*-pyrrole-2,4-dicarboxylic acid 2-tert-butyl ester 4-ethyl ester **1a** (30 g, 0.11 mol) was dissolved in 300 mL of tetrahydrofuran under stirring, followed by addition of 360 mL of acetic acid and 300 mL of H₂O. The mixture was homogeneously stirred and added with ammonium cerium nitrate (246 g, 0.45 mol) in one portion. After stirring for 0.5 hour, the reaction mixture was poured into 800 mL of ice-water, stirred for another 0.5 hour and filtered. The filter cake was dried under vacuum to obtain the title compound 5-formyl-3-methyl-1*H*-pyrrole-2,4-dicarboxylic acid 2-tert-butyl ester 4-methyl ester **1b** (31.13 g, yield 98%) as a pale yellow solid. MS m/z (ESI): 282.0[M+1]

### Step 2

### 5-(2-Ethoxycarbonyl-vinyl)-3-methyl-1H-pyrrole-2,4-dicarboxylic acid 2-tert-butyl ester 4-methyl ester

5-Formyl-3-methyl-1*H*-pyrrole-2,4-dicarboxylic acid 2-tert-butyl ester 4-ethyl ester **1b** (23 g, 81.7 mmol) and (carbethoxymethylene)triphenylphosphorane (34.66 g, 99.4 mmol) were dissolved in 450 mL of tetrahydrofuran under stirring. After stirring for 12 hours, the reaction mixture was concentrated under reduced pressure and the resulting residue was purified by silica gel column chromatography with elution system B to obtain the title compound 5-(2-ethoxycarbonyl-vinyl)-3-methyl-1*H*-pyrrole-2,4-dicarboxylic acid 2-tert-butyl ester 4-ethyl ester **1c** (24 g, yield 84%) as a pale yellow solid.
MS m/z (ESI): 352.1 [M+1]

### Step 3

### 5-(2-Ethoxycarbonyl-ethyl)-3-methyl-1H-pyrrole-2,4-dicarboxylic acid 2-tert-butyl ester 4-ethyl ester

Under hydrogen atmosphere, 5-(2-ethoxycarbonyl-vinyl)-3-methyl-1*H*-pyl-role-2,4-dicar boxylic acid 2-tert-butyl ester 4-ethyl ester **1c** (24 g, 68.3 mmol) was dissolved in 180 mL of anhydrous ethanol under stirring, followed by addition of 2.44 g 10% Pd/C. After stirring for 12 hours, the reaction mixture was filtered. The filter cake was washed with a small amount of ethanol before the filtrate was collected and concentrated under reduced pressure to obtain the title compound 5-(2-ethoxycarbonyl-ethyl)-3-methyl-1*H*-pyrrole-2,4-dicarboxylic acid 2-tert-butyl ester 4-ethyl ester **1d** (23 g, yield 95%) as a white solid.
MS m/z (ESI): 354.4[M+1]

### Step 4

### 5-(2-Carboxy-ethyl)-3-methyl-1H-pyrrole-2,4-dicarboxylic acid 2-tert-butyl ester 4-ethyl ester

5-(2-Ethoxycarbonyl-ethyl)-3-methyl-1*H*-pyrrole-2,4-dicarboxylic acid 2-tert-butyl ester 4-ethyl ester **1d** (23.6 g, 66.8 mmol) was dissolved in 190 mL of tetrahydrofuran and 90 mL of methanol under stirring, followed by dropwise addition of lithium hydroxide solution (10M, 80 mL). After stirring for 1 hour, the reaction mixture was concentrated under reduced pressure. The resulting residue was adjusted to pH 2 with hydrochloric acid (2 M), filtered and the filter cake was dried under vacuum to obtain the title compound 5-(2-carboxy-ethyl)-3-methyl-1*H*-pyrrole-2,4-dicarboxylic acid 2-tert-butyl ester 4-methyl ester **1e** (24 g, yield 98%) as a white solid.
MS m/z (ESI): 326.1[M+1]

### Step 5

### 5-(3-Hydroxy-propyl)-3-methyl-1H-pyrrole-2,4-dicarboxylic acid 2-tert-butyl ester 4-methyl ester

5-(2-Carboxy-ethyl)-3-methyl-1*H*-pyrrole-2,4-dicarboxylic acid 2-tert-butyl ester 4-ethyl ester **1e** (9.75 g, 30 mmol) was dissolved in 90 mL of anhydrous tetrahydrofuran under stirring. A solution of BH₃ (1 M) in THF (90 mL) was added slowly to the reaction mixture between -10∼-5 °C. After stirring for 2∼3 hours at room temperature, the reaction mixture was concentrated under reduced pressure. The reaction mixture was added with 100 mL of saturated sodium bicarbonate solution and 100 mL of ethyl acetate, and extracted with ethyl acetate (100 mL×3). The organic extracts were combined, washed with saturated saline solution (100 mL), dried over anhydrous magnesium sulfate, filtered and concentrated to obtain the title compound 5-(3-hydroxy-propyl)-3-methyl-1*H*-pyrrole-2,4-dicarboxylic acid 2-tert-butyl ester 4-methyl ester **1f** (9.2 g, yield 98%) as a pale yellow oil.
MS m/z (ESI): 312.3[M+1]

### Step 6

### 5-(3-Methanesulfonyloxy-propyl)-3-methyl-1H-pyrrole-2,4-dicarboxylic acid 2-tert-butyl ester 4-ethyl ester

5-(3-Hydroxy-propyl)-3-methyl-1*H*-pyrrole-2,4-dicarboxylic acid 2-tert-butyl ester 4-ethyl ester **1f** (9.20 g, 30 mmol) was dissolved in 150 mL of dichloromethane under stirring, followed by addition of triethylamine (7 mL, 50 mmol) and methyl sulfonyl chloride (3.5 mL, 45 mmol) successively at -10 °C. After stirring for 4 hours, the reaction mixture was added with a small amount of ice-water, washed with 0.5 M hydrochloric acid (80 mL×2), saturated sodium carbonate solution (80 mL × 2) and saturated saline solution (80 mL) successively, dried over anhydrous magnesium sulfate, filtered and concentrated under reduced pressure to obtain the title compound 5-(3-methanesulfonyloxy-propyl)-3-methyl-1*H*-pyrrole-2,4-dicarboxylic acid 2-tert-butyl ester 4-methyl ester **1g** (11.4 g, yield 99%) as a brown oil.
MS m/z (ESI): 390.5[M+1]

### Step 7

### (R)-4-Oxiranylmethyl-morpholine

Morpholine **1h** (8.7 mL, 0.1 mol) was dissolved in 4.5 mL of *tert*-butanol under stirring, followed by addition of (*R*)-(-)-epichlorohydrin (8.1 mL, 0.1 mol) in an ice bath. Then the reaction mixture was warmed up to room temperature and stirred for 24 hours. A solution of *tert*-butanol potassium (1.67 M) in tetrahydrofuran (60 mL) was added dropwise to the reaction mixture at 10 °C. After stirring for 30 minutes, the reaction mixture was concentrated under reduced pressure. The residue was added with 50 mL of H₂O and extracted with dichloromethane (100 mL×2). The organic extracts were combined, washed with saturated saline solution (100 mL), dried over anhydrous magnesium sulfate, filtered. The filtrate was concentrated under reduced pressure to obtain the title compound (*R*)-4-oxiranylmethyl-morpholine 1i (12.7 g, yield 88.8%) as a yellow oil.
MS *m*/*z* (ESI): 144.4[M+1]

### Step 8

### (S)-1-Amino-3-morpholin-4-yl-propan-2-ol

(*R*)-4-Oxiranylmethyl-morpholine **1i** (6.3 g, 44 mmol) was dropped slowly into 450 mL of 25% aqueous ammonia in an ice bath. After stirring for 18 hours, the reaction mixture was concentrated under reduced pressure to obtain the title compound (*S*)-1-amino-3-morpholin-4-yl-propan-2-01 **1j** (7 g, yield 99%) as a white solid.
MS m/z (ESI): 161.1[M+1]

### Step 9

### (S)-5-[3-(2-hydroxy-3-morpholin-4-yl-propylamino)-propyl]-3-methyl-1H-pyrrole-2,4-dicarboxylic acid 2-tert-butyl ester 4-ethyl ester

5-(3-Methanesulfonyloxy-propyl)-3-methyl-1*H*-pyrrole-2,4-dicarboxylic acid 2-tert-butyl 1 ester 4-ethyl ester **1g** (1.13 g, 2.9 mmol) was dissolved in 5.6 mL of dichloromethane under stirring, followed by addition of (*S*)-1-amino-3-morpholin-4-yl-propan-2-ol **1j** (0.93 g, 5.8 mmol). After stirring for 12 hours, the reaction mixture was heated to 45°C for 14 hours. The reaction mixture was added with 15 mL of saturated saline solution and extracted with dichloromethane (20 mL×3). The organic extracts were combined and concentrated under reduced pressure. The resulting residue was purified by silica gel column chromatography with elution system A to obtain the title compound (*S*)-5-[3-(2-hydroxy-3-morpholin-4-yl-propylamino)-propyl]-3-methy]-1*H*-pyrrole-2,4-dicarboxylic acid 2-tert-butyl ester 4-ethyl ester **1k** (600 mg, yield 72.5%) as a colorless oil.
MS m/z (ESI): 454.2[M+1]

### Step 10

### (R)-5-(2-hydroxy-3-morpholin-4-yl-propyl)-3-methyl-5,6,7,8-tetrahydro-1H-pyrrolo[3,2-c]azepin-4-one

(*S*)-5-[3-(2-hydroxy-3-morpholin-4-yl-propylamino)-propyl]-3-methyl-1*H*-pyrrole-2,4-dicarboxylic acid 2-tert-butyl ester 4-methyl ester **1k** (580 mg, 1.28 mmol) was dissolved in 6 mL of toluene under stirring, followed by dropwise addition of a solution of trimethylaluminium (2 M) in toluene (1.9 mL) in an ice bath. The reaction mixture was heated to reflux for 24 hours. The reaction mixture was concentrated under reduced pressure, added with 20 mL of hydrochloric acid (6 M) and stirred for 20 minutes. The resulting reaction mixture was adjusted to pH 12 with sodium hydroxide solution (12 M) and extracted with dichloromethane (50 mL×2). The organic extracts were combined and concentrated under reduced pressure. The resulting residue was purified by silica gel column chromatography with elution system A to obtain the title compound (*R*)- 5-(2-hydroxy-3-morpholin-4-yl-propyl)-3-methyl-5,6,7,8-tetrahydro-1*H*-pyrrolo[3,2-c]azepin-4-one **1m** (300 mg, yield 57.6%) as a white solid.
MS m/z (ESI): 308.2[M+1]

### Step 11

### (R)-5-(2-hydroxy-3-morpholin-4-yl-propyl)-3-methyl-4-oxo-1,4,5,6,7,8-hexahydro-pyrrolo[3,2-c]azepine-2-carbaldehyde

Chlorine ethylene dimethyl ammonium chloride (130 mg, 0.98 mmol) was dissolved in 3 mL of dichloromethane under stirring, followed by addition of a solution of (*R*)-5-(2-hydroxy-3-morpholin-4-yl-propyl)-3-methyl-5,6,7,8-tetrahydro-1*H*-pyrrolo[3,2-c]azepin-4-one **1m** (300 mg, 0.98 mmol) in dichloromethane (2 mL) at 0 °C. After stirring for 20 minutes at room temperature, the reaction mixture was added with 10 mL of sodium hydroxide solution (12 M) and 10 mL of saturated saline solution successively, extracted with a mixed solvent of dichloromethane and methanol (100 mL×3, V/V=10/1). The organic extracts were combined, washed with 100 mL of saturated saline solution, dried over anhydrous magnesium sulfate, filtered and concentrated under reduced pressure. The resulting residue was purified by silica gel column chromatography with elution system A to obtain the title compound (*R*)-5-(2-hydroxy-3-morphoiin-4-yl-propyl)-3-methyl-4-oxo-1,4,5,6,7,8-hexahydro-pyrrolo[3,2-*c*]azepine-2-carbaldehyde **1n** (200 mg, yield 61%) as a white solid.
MS m/z (ESI): 336.2[M+1]

### Step 12

### (R,Z)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1H-pyrrolo[3,2-c]azepin-4-one

(*R*)-5-(2-hydroxy-3-morpholin-4-yl-propyl)-3-methyl-4-oxo-1,4,5,6,7,8-hexahydro-pyrro lo[3,2-*c*]azepine-2-carbaldehyde **1n** (50 mg, 0.15 mmol) was dissolved in 261 µL of ethanol under stirring, followed by addition of 5-fluoro-1,3-dihydro-indol-2-one (20 mg, 0.13 mmol) and piperidine (7.3 µL, 0.074 mmol). After stirring for 2 hours at 80 °C in dark, the reaction mixture was cooled to room temperature, filtered and dried under vacuum to obtain the title compound (*R,Z*)-2-(5-fluoro-2-oxo-1,2-dihydro-indol-3-ylidenemethyl)-5-(2-hydroxy-3-morp holin-4-yl-propyl)-3-methyl-5,6,7,8,-tetrahydro-1H-pyrrolo[3,2-*c*]azepin-4-one **1p** (40 mg, yield 57%) as a yellow solid.
MS m/z (ESI): 469.2[M+1]
¹H NMR(400MHz, *d*-DMSO, ppm): δ13.73(s, 1H), 10.91(s, 1H), 7.76∼7.78(m, 1H), 7.75(s, 1H), 6.91∼6.94(m, 1H), 6.84∼6.87(m, 1H), 4.72∼4.73(d, 1H), 3.90(m, 1H), 3.75∼3.79(dd, 1H), 3.57∼3.59(1, 4H), 3.38∼3.35(t, 2H), 3.14∼3.19(dd, 1H), 2.92∼2.95(t, 2H), 2.46(s, 3H), 2.42∼2.51(m, 4H), 2.29∼2.31(t, 2H), 2.08(m, 2H)

### Step 13

### (R,Z)-2-(5-fluoro-2-oxo-1,2-dihydro-indol-3-ylidenemethyl)-5-(2-hydroxy-3-morpholin-4-yl-propyl)-3-methyl-5,5,7,8-tetrahydro-1H-pyrrolo[3,2-c]azepin-4-one maleate

(*R*,*Z*)-2-fluoro-2-oxo-1,2-dihydro-indol-3-ylidenemethyl)-5-(2-hydroxy-3-ylidenethyl)-5-(2-hydroxy-3-morpholin-4-yl-propyl)-3-methyl-5,6,7,8-tetrahydro-1*H*-pyrrolo[3,2-c]azepin-4-one **1p** (731 mg, 1.56 mmol) and maleic acid (217 mg, 1.87 mmol) was dissolved in 150 mL of methanol under stirring. After stirring for 20 minutes at 40°C, the reaction mixture was filtered and the filtrate was concentrated under reduced pressure. The reaction mixture was added with 50 mL of acetonitrile and heated to reflux for 20 minutes. The reaction mixture was cooled to room temperature, filtered to obtain (*R*,*Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-c]azepin-4 -one maleate **1** (831 mg, yield 91.1%) as a yellow solid.
MS m/z (ESI): 469.2[M+1]
¹H NMR(400MHz, *d*-DMSO, ppm): δ13.76(s, 1H), 10.93(s, 1H), 7.77∼7,80(m, 1H), 7.76(s, 1H), 6.93∼6.98(m, 1H), 6.85∼6.88(m, 1H), 6.05(s, 2H), 4.19(d, 1H), 3.63∼3.84(m, 4H), 3.60∼3.61(m, 1H.), 3.43∼3.46(m, 4H), 3.31(m, 2H), 3.13∼3.18(m, 3H), 2.96∼3.011(m, 3H), 2.48(s, 3H), 2.10∼2.13(m, 2H)

### Example 2

### (R,Z)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1H-pyrrolo[3,2-c]azepin-4-one malate

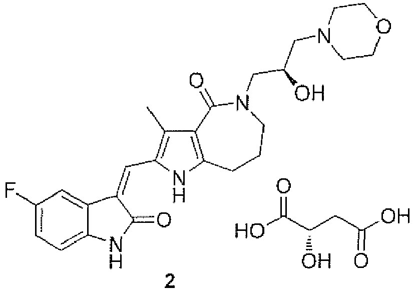

(*R,Z*)-2-(5-fluoro-2-oxo-1,2-dihydro-indol-3-ylidenemethyl)-5-(2-hydroxy-3-mor-pholin-4-yl-propyl)-3-methyl-5,6,7,8-tetrahydro-1*H*-pyrrolo-[3,2-c]azepin-4-one **1p** (469 mg, 1 mmol) was dissolved in 15 mL of methanol under stirring, followed by addition of L-malic acid (147 mg, 1.1 mmol). After stirring for 30 minutes, the reaction mixture was concentrated under reduced pressure, added with 120 mL of acetonitrile and heated to reflux for 1.5 hours. The reaction mixture was cooled to room temperature and filtered. The filter cake was washed with acetonitrile (1 mL×3) and ethanol (1 mL×3) (both ice-cooled) successively to obtain (R,Z)-2-(5-fluoro-2-oxo-1,2-dihydro-indol-3-ylidenemethyl)-5-(2-hydroxy-3-morpholin-4-y]-propyl)-3-methyl-5,6,7,8-tetrahydro-1*H*-pyrrolo[3,2-*c*]azepin-4-one malate **2** (535 mg, yield 88.8%) as a yellow solid.
MS m/z (ESI): 469.2[M+1]
¹H NMR(400MHz, *d*-DMSO, ppm): δ13.73(s, 1H), 10.92(s, 1H), 7.75∼7.79(m, 2H), 6.83∼6.96(m, 2H), 4.91(s, 1H), 4.17∼4.20(m, 1H), 3.95(m, 1H), 3.72∼3.77(dd, 1H), 3.61(s, 3H), 3.20∼3.37(m, 4H), 2.92∼2.96(m, 2H), 2.39∼2.62(m, 14H), 2.25(m, 2H)

### Example 3

### (R,Z)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1H-pyrrolo[3,2-c]azepin-4-one lactate

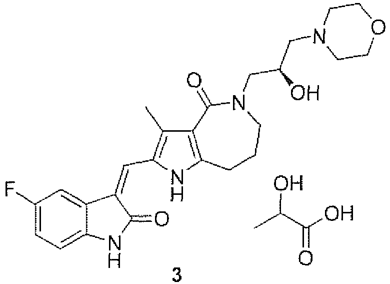

(*R,Z*)-2-(5-fluora-2-oxa-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-*c*]azepin-4-one **1p** (471 mg, 1 mmol) was dissolved in 17 mL of methanol and 34 mL of dichloromethane under stirring, followed by addition of lactic acid (90 mg, 1 mmol). After stirring for 30 minutes, the reaction mixture was concentrated under reduced pressure, added with 20 mL of acetonitrile and heated to reflux for 45 minutes. The reaction mixture was cooled to room temperature and filtered. The filter cake was washed with acetonitrile (1 mLx3) and ethanol (1 mLx3) (both ice-cooled) successively to obtain (*R,Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2*-c*] azepin-4-one lactate **3** (502 mg, yield 90%) as a yellow solid.
MS m/z (ESI): 469.2[M+1]
¹H NMR(400MHz, *d*-DMSO, ppm): δ13.73(s, 1H), 10.92(s, 1H), 7.75∼7.79(m, 2H), 6.92∼6.94(m, 1H), 6.84∼6.87(m, 1H), 4.74(d, 1H), 3.79∼3.90(m, 1H), 3.75(dd, 1H), 3.59(s, 3H), 3.58(m, 1H), 3.32(m, 2H), 3.19(m, 1H), 2.95(m, 2H), 2.46(m, 4H), 2.45(m, 8H), 2.33(m, 2H), 2.26(m, 2H)

### Example 4

### (R,Z)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1H-pyrrolo[3,2-c]azepin-4-one mesylate

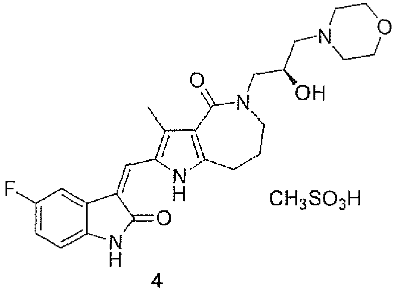

(*R,Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-*c*]azepin-4-one **1p** (470 mg, 1 mmol) was dissolved in 8 mL of methanol and 16 mL of dichloromethane under stirring, followed by addition of methanesulfonic acid (96 mg, 1 mmol). After stirring for 30 minutes, the reaction mixture was concentrated under reduced pressure, added with 10 mL of acetonitrile and heated to reflux for 30 minutes. The reaction mixture was cooled to room temperature and filtered. The filter cake was washed with acetonitrile (1 mL×3) and ethanol (1 mL×3) (both ice-cooled) successively to obtain (*R,Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H-*pyrrolo[3,2-*c*]azepin-4-one mesylate **4** (519 mg, yield 92%) as a yellow solid.
MS m/z (ESI): 469.2[M+1]
¹H NMR(400MHz, *d*-DMSO, ppm): δ13.77(s, 1H), 10.94(s, 1H), 9.66(s, 1H), 7.76∼7.80(m, 2H), 6.85∼6.97(m, 2H), 5.82(s, 1H), 4.22(s, 1H), 4.00(m, 2H), 3.83(m, 3H), 3.77(m. 2H), 3.58∼3.61(m, 3H), 3.21∼3.35(m, 4H), 3.13(m, 2H), 2.51(s, 3H), 2.33(s, 3H), 2.10(m, 2H), 1.1(m, 2H)

### Example 5

### (R,Z)-2-(5-fluoro-1,2-oxo-dihydro-indol-3-ylidenemethyl)-5-(2-hydroxy-3-morpholin-4-yl-propyl)-3-methyl-5,6,7,8-tetrahydro-1H-pyrrolo[3,2-c]azepin-4-one hydrochloride

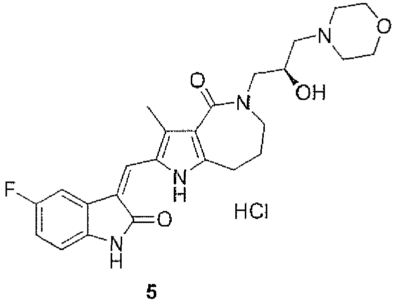

(*R,Z*)-2-(5-fluoro-2-oxo-1,2-dihydro-indol-3-ylidenemethyl)-5-(2-hydroxy-3-morpholoin-4-yl-propyl)-3-methyl-5,6,7,8-tetrahydro-1*H-*pyrrolo-[3,2-*c*]azepin-4-one **1p** (486 mg, 1 mmol) was dissolved in 5 mL of methanol and 8 mL of dichloromethane under stirring, followed by addition of a solution of hydrogen chloride (5M) in 1,4-dioxane (2 mL). After stirring for 1 hour, the reaction mixture was concentrated under reduced pressure, added with 50 mL of acetonitrile and heated to reflux for 1 hour. The reaction mixture was cooled to room temperature and filtered to obtain (*R,Z*)-2-(5-fluoro-2-oxo-1,2-dihydro-indol-3-ylidenemthyl-5-(2-hydroxy-3-morpholin-4-yl-propyl)-3-methyl-5,6,7,8-tetrahydro-1*H*-pyrrolo[3,2-c]azepin -4-one hydrochloride **5** (459 mg, yield 91%) as a yellow solid.
MS m/z (ESI): 469.2[M+1]
¹H NMR(400MHz, *d*-DMSO, ppm): δ13.73(s, 1H), 10.91(s, 1H), 7.76∼7.78(m, 1H), 7.75(s, 1H), 6.91∼6.94(m, 1H), 6.84∼6.87(m, 1H), 4.72∼4.73(d, 1H), 3.90(m, 1H), 3.75∼3.79(dd, 1H). 3.57∼3.59(t, 4H), 3.38̃∼3.35(t, 2H), 3.14∼3.19(dd, 1H), 2.92∼2.95(t, 2H), 2.46(s, 3H), 2.42∼2.51(m, 4H), 2.29∼2.31(t, 2H), 2.08(m, 2H)

### TEST EXAMPLES

### SOLUBILITY ASSAY

According to the conventional solubility measurement, the solubility of the compound of formula (1) and salts thereof were determined in physiological saline. The results were shown in table 1:

**Table 1:**

| The form of salt | Physiological saline |
|---|---|
| The compound of formula (I) | 0.00186 |
| Example 1 | 0.1214 |
| Example 2 | 0.0227 |
| Example 3 | 0.00124 |
| Example 4 | 0.0131 |
| Example 5 | 0.0294 |

Conclusion: Compared with free base and other salts of the compound of formula (1), the solubility of maleate of the compound of formula (I) has significantly improved.

### PHARMACOKINETICS ASSAY

### Test Example 1: Pharmacokinetics assay of the compounds of the present invention

### 1. Experimental purpose

The rats were used as experimental animals. The compound of formula (I) and other salts thereof were administrated intragastrically, the maleate of the compound of formula (I) was injected into the tail vein to determine the drug concentration in plasma at different time points by LC/MS/MS method. The pharmacokinetic behavior, characteristics and the oral absolute bioavailability of the compounds in the present invention were studied and evaluated in rats.

### 2. Protocol

### 2.1 Experimental samples

The compound of formula (I), compounds of Example 1∼5.

### 2.2 Experimental animals

28 healthy adult SD rats, male and female in half, were divided into 7 groups (4 rats in each group) and purchased from SINO-BRITSH SIPPR/BK LAB. ANIMAL LTD., CO, License number: SCXK (Shanghai) 2008-0016.

### 2.3 Equipments

TSQ Quantum Ultra AM triple quadrupole mass spectrometer, Thermo Finnigan (American);
Agilent 1200 high performance liquid chromatography system, Agilent (American).

### 2.4 Preparation of the tested compounds

The intravenous injection administration group: the suitable amount of compounds were weighted, added into physiological saline and diluted to the final volume. The sample concentration was 1.0 mg/mL.

The intragastrical administration group: the suitable amount of compounds were weighted and added into 0.5% CMC-Na to prepare 1.0 mg/mL suspension by using ultrasound device. The sample should be prepared freshly away from light at the time of use.

### 2.5 Administration

28 healthy adult SD rats, male and female in half, were divided into 7 groups (4 rats in each group). After an overnight fast, the compound of Example 1, at a dose of 10 mg/kg (calculated by the base part), at a volume of 10 mL/kg, was administered intragastrically or injected into the tail vein.

### 2.6 Sample Collection

For the intravenous injection administration group, blood samples (0.2 mL) were taken from orbital sinus at pre administration and at 2 minutes, 15 minutes, 30 minutes, 1.0 hour, 2.0 hours, 4.0 hours, 6.0 hours, 8.0 hours, 12.0 hours, 24.0 hours and 36.0 hours post administration, stored in heparinized tubes and centrifuged for 10 minutes at 3,500 rpm. The plasma samples were stored at -20°C.

For the intragastrical administration group, blood samples were taken at pre administration and at 0.5, 1.0, 2.0, 3.0, 4.0, 6.0, 5.0, 12.0, 24.0 and 36.0 hours post administration. The method to treat the samples was the same with the intravenous injection administration group. The rats were fed 2 hours after administration.

### 2.7 Analytical method

25 µL of rat plasmas taken at various time points after administration were mixed with 20 µL of internal standard solution and 125 µL of methanol for 2 minutes by using a vortexer and the mixture was centrifuged for 10 minutes at 16,000 rpm. 10 µL of the supernatant was analyzed by LC-MS/MS.

### 2.8 Preparation of the standard curve

25 µL of rat blank plasmas were mixed with a series of standard solutions respectively to obtain plasma concentration of 1.00, 2.00, 5.00, 25.0, 100, 500, 2000 and 5000 ng/mL. 20 µL of internal standard solution and 150 µL of methanol were added and then the mixture was operated according to "plasma sample pretreatment" method. Plasma concentration was used as the abscissa, the ratio of chromatographic peak area between the sample and internal standard was used as the ordinate, the linear regression was carried out by the weighted least square method (w=1/x²) to obtain the typical standard curve equation.

### 2.9 Calculation of the pharmacokinetic parameters

Compartment model fitting was carried out on the pharmacokinetic behavior of the tested compounds to calculate the main pharmacokinetic parameters, wherein the measured values were taken for Cₘₐₓ and tₘₐₓ. Oral absolute bioavailability was calculated according to AUC₀₋ₜ after intragastrical administration and intravenous injection at tail vein.

### 3. Results of pharmacokinetic parameters

Pharmacokinetic parameters of the compounds of the present invention were shown as table 2.

Conclusion: Compared with free base and other salts of the compound of formula (I), maleate of the compound of formula (I) had significant improvement in pharmacokinetic characteristics and bioavailability, and had obvious pharmacokinetic advantage.

**Table 2:**

| Compound | *F* (%) | *C*ₘₐₓ (µg/mL) | *A UC₀₋₁* (µg-h/mL) | *t 1*/*2* (h) | *Tₘₐₓ* (h) | MRT (h) | CL/F (mL/min/kg) |
|---|---|---|---|---|---|---|---|
| The compound of formula (I) | 27.9 | 0.64 ± 0.24 | 4.49 ±2.79 | 3.31 ± 0.57 | 2.00 ± 1.41 | 5.24 ± 2.23 | 1.84 ± 1.20 |
| | | Vein | 16.12 ± 3.97 | 5.97 ± 2.50 | — | 1.40 ± 0.35 | 0.65 ± 0.161 |
| Example 1 | 48.2 | 0.95 ±0.37 | 7.77 ± 2.99 | 3.01 ± 0.60 | 5.75 ± 2.06 | 6.66 ± 2.05 | 1.44 ± 0.55 |
| | | Vein | 16.12 ±3.97 | 5.97 ± 2.50 | — | 1.40 ± 0.35 | 0.65 ± 0.161 |
| Example 2 | 30.4 | 0.85 ± 0.39 | 4.90 ± 2.79 | 3.13 ± 0.97 | 1.75 ± 1.66 | 4.29 ± 1.87 | 1.79 ± 0.67 |
| | | Vein | 16.12 ±3.97 | 5.97 ± 2.50 | — | 1.40 ± 0.35 | 0.65 ± 0.161 |
| Example 3 | 36.6 | 0.81 ± 0.45 | 5.90 ± 3.35 | 3.06 ± 1.21 | 2.50 ± 1.00 | 6.28 ±1.56 | 1.64 ± 0.70 |
| | | 36.6 Vein | 16.12 ±3.97 | 5.97 ± 2.50 | — | 1.40 ± 0.35 | 0.65 ± 0.161 |
| Example 4 | 45.3 | 0.77 ± 0.39 | 7.30 ± 5.87 | 3.41 ± 0.85 | 3.75 ± 1.71 | 6.68 ± 1.58 | 1.49 ± 0.69 |
| | | Vein | 16.12 ±3.97 | 5.97 ±2.50 | — | 1.40 ± 0.35 | 0.65 ± 0.161 |
| Example 5 | 25.2 | 0.50 ± 0.10 | 4.07 ±1.63 | 3.60 ± 0.52 | 4.25 ± 2.06 | 6.80 ± 1.58 | 2.03 ± 0.51 |
| | | Vein | 16.12 ± 3.97 | 5.97 ± 2.50 | — | 1.40 ± ().35 | 0.65 ± 0.161 |

## Claims

1. A pharmaceutically acceptable salt of (*R,Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H*-pyrrolo[3,2-*c*]azepin-4-one of formula (I):

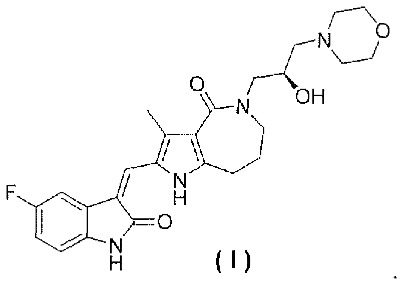

2. The salt according to claim 1, wherein the salt is an inorganic salt.

3. The salt according to claim 2, wherein the inorganic salt is selected from the group consisting of phosphate, hydrochloride, sulfate, nitrate and hydrobromide, preferably hydrochloride.

4. The salt according to claim 1, wherein the salt is an organic salt.

5. The salt according to claim 4, wherein the organic salt is selected from the group consisting of mesylate, maleate, tartrate, succinate, acetate, trifluoroacetate, fumarate, citrate, benzene sulfonate, benzoate, naphthalene sulphonate, lactate and malate; preferably malate, lactate, mesylate or maleate, most preferably maleate.

6. A method of preparing the salt according to any one of claims 1. to 5, wherein the method comprises a step of reacting (*R,Z*)-2-(5-fluoro-2-oxo-1,2-dihydro-indol-3-ylidenemethyl)-5-(2-hydroxy-3-morpholin-4-yl-propyl)-3-methyl-5,6,7,8-tetrahydro-1*H* -pyrrolo[3,2-*c*]azepin-4-one with a corresponding acid to prepare the salt.

7. The method according to claim 6, wherein the acid is an inorganic acid/organic acid selected from the group consisting of phosphoric acid, hydrochloric acid, sulfuric acid, nitric acid, hydrobromic acid, methanesulfonic acid, maleic acid, tartaric acid, succinic acid, acetic acid, trifluoroacetic acid, fumaric acid, citric acid, benzenesulfonic acid, benzoic acid, naphthalenesulfonic acid, lactic acid and malic acid.

8. A pharmaceutical composition comprising a therapeutically effective amount of the salt according to any one of claims 1 to 5 and a pharmaceutically acceptable carrier.

9. A use of the salt according to any one of claims 1 to 5 or the pharmaceutical composition according to claims 8 in the preparation of a medicament for the treatment of a protein kinase related disease.

10. The use according to claim 9, wherein the protein kinase related disease is selected from the group consisting of a disease related with VEGFR-2, EGFR, HER-2, PDGFR, c-Kit, c-Met and FGFR.

11. The use according to claim 10, wherein the disease is cancer.

12. The use according to claim 11, wherein the cancer is selected from the group consisting of lung cancer, breast cancer, epidermal squamous cell carcinoma and gastric cancer.

13. A use of the salt according to any one of claims 1 to 5 or the pharmaceutical composition according to claim 8 in the preparation of a protein kinase inhibitor drug, wherein the protein kinase is selected from the group consisting of VEGFR-2, EGFR, HER-2, PDGFR, c-Kit, c-Met and FGFR.
